# EUROPÄISCHE PATENTSCHRIFT

(11) **EP 0 540 037 B1**
(45) Veröffentlichungstag und Bekanntmachung des Hinweises auf die Patenterteilung: **18.12.1996**
(21) Anmeldenummer: 92118637.5
(22) Anmeldetag: 30.10.1992
(51) Int. Cl.: G01N 33/543, G01N 33/58

(54) **Verfahren zur Bestimmung einer immunologisch bindefähigen Substanz**
Method for the determination of an immunologically bindable substance
Procédé de détermination d'une substance apte à se lier immunologiquement

(30) Priorität: 31.10.1991 DE 4136010
(43) Veröffentlichungstag der Anmeldung: 05.05.1993
(73) Patentinhaber: BOEHRINGER MANNHEIM GMBH, 68298 Mannheim (DE)
(72) Erfinder: Deger, Arno, Dr., W-8124 Seeshaupt (DE); Uhl, Wolfgang, Dr., W-8120 Weilheim (DE)
(74) Vertreter: Huber, Bernhard, Dipl.-Chem.

(56) Entgegenhaltungen:
- EP-A- 0 073 514
- EP-A- 0 160 900
- US-A- 5 164 299

## Beschreibung

Die Erfindung betrifft ein Verfahren zur Bestimmung einer immunologisch bindefähigen Substanz.

Die Nutzung der hohen Bindungsstärke zwischen Streptavidin und Biotin zum Aufbau immunologischer Testverfahren ist bereits in vielfacher Form realisiert. Auf inertem Material (Polystyrolkugeln, -tubes etc.) immobilisiertes Streptavidin dient als universelle Festphase mit hoher Bindekapazität für das entsprechende biotinylierte Reagenz. Gut reproduzierbare Beschichtungssverfahren und ausgezeichnete Stabilitäten der Streptavidinmatrix bilden die Basis für die hohen Präzisionen, die mit dieser Technologie erreicht werden.

Trotz der beschriebenen hohen Qualität von Streptavidin-Festphasen sind Inhomogenitäten und Störungen, wie insbesondere sogenannte "Blutungseffekte" durch Ablösung des Festphasengebundenen Streptavidins von der Membran, nicht vollständig auszuschließen. Entsprechend empfindliche Tests können darauf mit deutlichen Performanceeinbußen reagieren. Die Nachteile gegenüber direkt beschichteten Trägern werden besonders deutlich bei
1-Schritt-Sandwich-Assays mit nur einem Antikörper als Basis für Fänger- und Markerkomponente. Die zu wählende Stöchiometrie hinsichtlich der beiden Immunreagenzien repräsentiert ein auf die im Testansatz ablaufenden Reaktionen fein abgestimmtes Konzentrationsverhältnis. Nicht immer existieren "Plateaubereiche" für die biotinylierte Komponente, durch die Störungen geringeren Umfangs kompensiert werden. Entsprechend kritisch sind Stabilitätsprobleme und Produktionsschwankungen in diesen Fällen einzustufen.

Die Aufgabe der vorliegenden Erfindung war es daher, ein Verfahren bereitzustellen, bei dem derartige Ausblutungseffekte das Testergebnis nicht beeinträchtigen.

Gelöst wird die Aufgabe erfindungsgemäß durch ein Verfahren zur Bestimmung eines Analyten durch einen heterogenen immunologischen Test unter Verwendung eines an einer Festphase fixierten ersten Partners eines Bindepaares, bei dem man eine den Analyten enthaltende Probelösung in Gegenwart der Festphase entweder
(1) mit einem ersten Konjugat, bestehend aus (a) dem zweiten Partner des oben erwähnten Bindepaares und (b) einer ersten, mit dem Analyten immunologisch spezifisch bindefähigen Substanz, oder
(2) mit einem ersten Konjugat, bestehend aus (a) dem zweiten Partner des oben erwähnten Bindepaares und (b) einer ersten, mit dem Analyten immunologisch spezifisch bindefähigen Substanz, sowie mit einer zweiten, mit dem Analyten immunologisch spezifisch bindefähigen markierten Substanz
inkubiert, danach die Phasen trennt, im Falle (1) die flüssige Phase durch eine weitere Lösung, die eine zweite, mit dem Analyten spezifisch bindefähige markierte Substanz enthält, ersetzt und erneut inkubiert und danach abtrennt, und in beiden Fällen die Markierung an der festen Phase oder in der zuletzt abgetrennten flüssigen Phase als Maß für die Analytmenge mißt, welches dadurch gekennzeichnet ist, daß man der flüssigen Phase vor der Inkubation außerdem ein zweites Konjugat aus (a) einem Makromolekül, das nicht mit dem Analyten bindefähig ist und (b) dem zweiten Partner des oben erwähnten Bindepaares im Überschuß auf das erste Konjugat zusetzt.

Für erfindungsgemäße Verfahren sind daher heterologe Immunoassays geeignet, bei denen zuerst die eigentliche Immunreaktion zwischen Analyt und dem ersten Konjugat stattfindet, dann der dabei gebildete Komplex aus Analyt und immunologisch spezifisch bindefähiger Substanz an einer Festphase immobilisiert wird und schließlich nach Abtrennung der flüssigen Phase zur Festphase eine neue flüssige Phase zugegeben wird, die dann über die Markierung einer darin enthaltenen zweiten, Analyt-spezifischen Substanz den Nachweis des Analyten ermöglicht (Fall 1). Desgleichen wird jedoch auch eine heterologe Verfahrensführung ermöglicht, bei der in der ersten flüssigen Phase sowohl das Konjugat, das aus (a) der ersten, mit dem Analyt immunologisch spezifisch bindefähige Substanz und (b) dem zur Bindung an die Festphase fähigen Partner besteht, als auch die zweite, immunologisch spezifisch bindefähige markierte Substanz (Fall 2) enthalten ist. Dabei wird in einer Flüssigkeitsphase der Analyt gleichzeitig markiert und an die Festphase gebunden und kann dann in immobilisiertem Zustand nach Entfernung überschüssiger Markierung mit der flüssigen Phase direkt bestimmt werden.

Durch die erfindungsgemäße Zugabe eines zweiten Konjugats aus (a) einem Makromolekül, das nicht mit dem Analyten bindefähig ist, und (b) einer an die Festphase bindefähigen Substanz werden die durch Ausbluten der Festphase abgelösten Festphasenbindepartner neutralisiert und können den Ablauf der immunologischen Bestimmung nicht beeinträchtigen. Überraschenderweise wurde im Rahmen der Erfindung außerdem festgestellt, daß eine deutlich verbesserte Qualität der Eichkurve und damit der Empfindlichkeit zu beobachten ist. Einem nur geringfügigen Signalanstieg beim untersten Standard (10 bis 20 mE) steht eine um etwa 500 mE erhöhte Extinktion beim obersten Standard gegenüber. Von besonderem Vorteil ist die damit verbundene Optimierung der Differenzierbarkeit im Bereich sehr niedriger Konzentrationen. Die generell zunehmende Steilheit der Eichkurve führt bei gleicher Signalpräzision zwangsläufig zu niedrigeren Konzentrationsvariations-Koeffizienten. Möglicherweise ist dies dadurch verursacht, daß bereits abgelöste Festphasenpartner über das zweite Konjugat, das vorzugsweise mehrere Bindungsstellen für den Festphasenpartner aufweist, wieder an die Festphase gebunden werden. Konjugate mit mehreren Bindungsstellen für den Festphasenpartner werden durch Umsetzen des Makromoleküls mit dem anderen Bindepartner in einem molaren Überschuß des Bindepartners, vorzugsweise von 5 : 1 bis 10 : 1 hergestellt.

Erneut überraschend zeigt sich bereits auf Signalebene ein auf ca. 50 % gesunkener Variationskoeffizient, der vermutlich auf die stärkere Absättigung der Festphasenwand zurückzuführen ist.

Vorzugsweise wird im Rahmen des erfindungsgemäßen Verfahrens als immunologisch bindefähige Substanz ein Antikörper oder auch ein Antikörperfragment verwendet. Vorzugsweise werden für das erfindungsgemäße Verfahren monoklonale Antikörper oder Fragmente davon (z.B. F(ab')₂-, Fab- oder Fab'-Fragmente) eingesetzt. Es kann aber ebenso ein polyklonales Antiserum verwendet werden.

Als spezifisches Bindepaar wird im Rahmen der Erfindung vorzugsweise Biotin/Streptavidin verwendet, wobei wiederum bevorzugt das Streptavidin an die Festphase gebunden ist und Biotin an die in der homogenen Phase vorhandenen Substanzen, nämlich die spezifisch bindefähigen Substanz ebenso wie an die unspezifische Substanz gebunden ist. Beispielsweise kann erfindungsgemäß auch ein Bindungspaar bestehend aus Hapten/spezifischer Antikörper verwendet werden, wobei als Hapten vorzugsweise Digoxin eingesetzt wird.

Das erfindungsgemäße Verfahren ermöglicht es, eine Reduktion der in der Flüssigphase vorhandenen Biotin-Kapazität, die durch von der Festphase abgelöstes Streptavidin verursacht wird, das merkliche Mengen der biotinylierten und mit dem Analyt spezifisch bindefähigen Substanz neutralisiert, zu verhindern. Die erfindungsgemäße Verfahrensführung läßt sich auf alle immunologischen Tests anwenden, bei denen in heterologen Immunoassays mit Hilfe eines spezifischen Bindepaares die letztendliche Bindung des Analyts an die feste Phase bewirkt wird.

Im Rahmen der Erfindung kann als Konjugat aus Makromolekül und dem zweiten Partner des Bindepaares vorzugsweise ein Konjugat verwendet werden, das als Makromolekül ein Protein, das gegebenenfalls durch Zucker-, Phosphat- oder/und Lipidreste modifiziert sein kann, z.B. einen unspezifischen Antikörper, d.h. einen Antikörper (MG ∼ 150.000), der mit keiner Komponente des Testsystems eine spezifische Bindung zeigt, oder ein anderes Protein, wie z.B. Albumine (MG 68000) wie Rinderserumalbumin oder aggregiertes Albumin (MG > 1 Mio., vgl. EP-A 0 269 092), enthält. Weitere geeignete Makromoleküle sind z.B. Kohlehydrate, Lipide, synthetische Polymere und Nukleinsäuren. Vorzugsweise liegt das Molekulargewicht der Makromoleküle im Bereich von 50.000 bis 2 Mio. Die Herstellung der erfindungsgemäßen Konjugate erfolgt auf an sich bekannte Weise, vorzugsweise durch Reaktion des aktivierten Bindepartners (z.B. eines reaktiven Biotinderivats oder eines reaktiven Haptenderivats, z.B. Digoxinderivats) mit reaktiven Gruppen (z.B. NH₂- oder SH-Gruppen) auf dem Makromolekül. Die Herstellung von reaktiven Biotin- und Haptenderivaten ist in JACS 100 (1978), 3585-3590 beschrieben.

Ein weiterer Gegenstand der Erfindung ist ein Reagenz zur Bestimmung eines Analyten durch einen heterogenen immunologischen Test unter Verwendung eines an einer Festphase fixierten ersten Partners eines Bindepaares,
das ein erstes Konjugat, bestehend aus (a) dem zweiten Partner des oben erwähnten Bindepaares und (b) einer ersten, mit dem Analyten immunologisch spezifisch bindefähigen Substanz, sowie eine zweite, mit dem Analyten immunologisch spezifisch bindefähige markierte Substanz enthält, welches dadurch gekennzeichnet ist, daß es weiterhin ein zweites Konjugat, bestehend aus (a) einem Makromolekül, das nicht mit dem Analyten bindefähig ist, und (b) dem zweiten Partner des oben erwähnten Bindepaares enthält.

Vorzugsweise ist das im erfindungsgemäßen Reagenz enthaltene zweite Konjugat aus (a) einem Makromolekül, das nicht mit dem Analyten bindefähig ist und (b) dem zweiten Partner des Bindepaares ein Konjugat, das mehrere Bindungsstellen für den ersten Partner des Bindepaares, der an der Festphase immobilisiert ist, aufweist.

Bei dem erfindungsgemäßen Reagenz wird als Bindepaar vorzugsweise Streptavidin/Biotin verwendet. Das im erfindungsgemäßen Reagenz enthaltene Makromolekül ist vorzugsweise ein Protein, besonders bevorzugt ein unspezifischer Antikörper oder Rinderserumalbumin.

Weiterhin ist es bei dem erfindungsgemäßen Reagenz bevorzugt, daß das zweite Konjugat aus (a) einem Makromolekül, das nicht mit dem Analyten bindefähig ist, und (b) dem zweiten Partner des Bindepaares mehrere Bindungsstellen für den ersten Partner des Bindepaares aufweist.

Der in den Beispielen verwendete monoklonale Antikörper gegen Creatinkinase vom Typ MM wurde bei der European Collection of Animal Cell Cultures (ECACC), Porton Down, GB-Salisbury, Wiltshire SP4 OJG unter der Hinterlegungsnummer ECACC 88091404 hinterlegt.

Die folgenden Beispiele sollen die Erfindung weiter erläutern.

### Abkürzungen:

- CK MM:: Creatinkinase vom Typ MM
- RSA:: Rinderserumalbinum
- PEG 40.000:: Polyethylenglycol (MG:40.000)
- R-IgG:: Rinder-Immunglobulin G
%-Angaben sind Gew.-%-Angaben.

### Beispiel 1

### Biotinylierung von Antikörpern

Monoklonale Antikörper gegen CK MM (ECACC 88091404) oder gegen CA 125 (Boehringer Mannheim GmbH, Katalog Nr. 11 22 789) werden gemäß JACS 100 (1978) 3585 - 3590 mit D-Biotinyl-ε-aminocapronsäure-N-hydroxysuccinimidester im Verhältnis 7:1 (Biotin: IgG) umgesetzt.

### Beispiel 2

### Biotinylierung von Rinderserumalbumin (RSA) zu RSA-Biotin

Zur Biotinylierung wird RSA gemäß JACS 100 (1978), 3585 - 3590 mit D-Biotinyl-ε-aminocapronsäure-N-hydroxysuccinimidester im Verhältnis 1:5 oder 1:10 (RSA:Biotin) umgesetzt.

### Beispiel 3

### Bestimmung von CA 125 (Einschrittverfahren)

10 µg/ml nach Beispiel 1 hergestellter biotinylierter MAK gegen CA 125, gegebenenfalls 50 µg/ml biotinylierter MAK gegen CKMM oder 20 - 50 µg/ml biotinyliertes RSA (Beispiel 2) sowie ein Konjugat aus Peroxidase und MAK gegen CA 125 (Peroxidaseaktivität ca. 30 U/ml) werden mit Inkubationspuffer im Verhältnis 1:1:1:100 zur Arbeitslösung AL gemischt.

### Inkubationspuffer (pH 7,4):

| Substanz | Konzentration |
|---|---|
| Na-Phosphat | 40 mmol/l |
| Di-Na-tartrat | 200 mmol/l |
| Pluronic® F 68 | 0.6 % |
| Phenol | 0.01 % |
| RSA | 0.2 % |
| PEG 40.000 | 0.75 % |
| R-IgG | 0.1 % |

- Waschlösung:: 250 mg NaCl und 1 mg CuSO₄ in 1 l H₂O dest

100 µl Probe werden mit 1000 µl AL in mit Streptavidin beschichteten Polystyrolröhrchen (Herstellung nach EP-A 0 269 092) 180 min. inkubiert. Die Röhrchen werden ausgesaugt und zweimal mit Waschlösung gewaschen. Anschließend wird 1 ml Substratlösung (100 mmol/l Phosphat-Citrat-Puffer pH 5,0, 1,47 mmol/l Natriumperborat, 9,1 mmol/l 2,2'Azino-di-[3-ethyl-benzthiazolin-sulfonsäure(6)] diammoniumsalz) zugegeben 60 min inkubiert und die gebildete Farbe bei 420 nm photometrisch bestimmt. Die Ergebnisse sind aus den Tabellen I bis VII zu ersehen.

Erläuterungen zu den Tabellen:
- MAK CK-Bi:: Konjugat aus MAK gegen CK MM mit Biotin (unspezifisches Konjugat)
- MAK CA 125-Bi:: Konjugat aus MAK gegen CA 125 mit Biotin (Analyt-bindendes Konjugat)
- a - e:: CA 125-Standards
Konzentrationen von ca. 0 - 500 U/ml

Die Zahlenwerte der Tabellen sind jeweils Extinktionswerte gemessen bei 420 nm in mE (Tabelle VII: E).

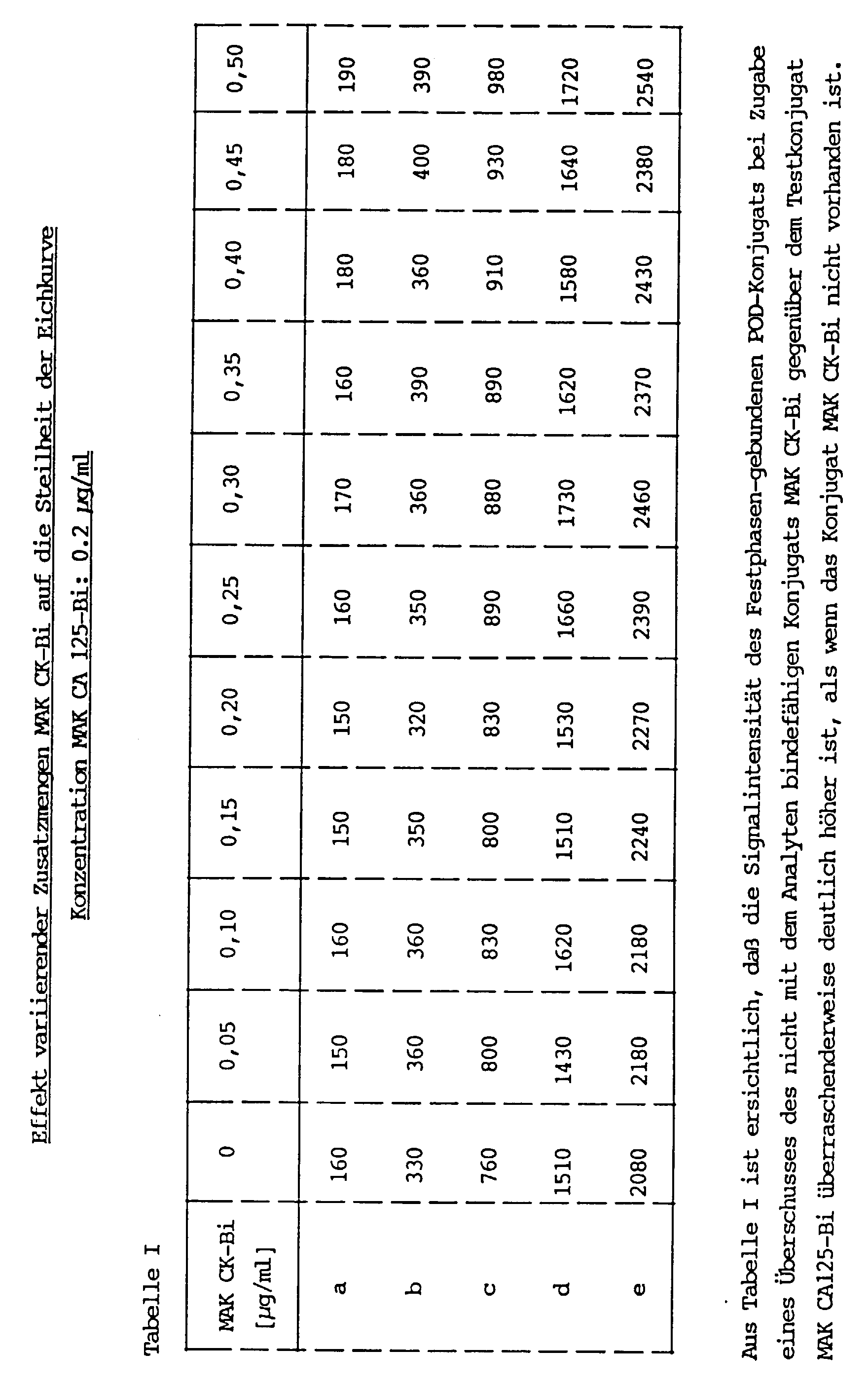

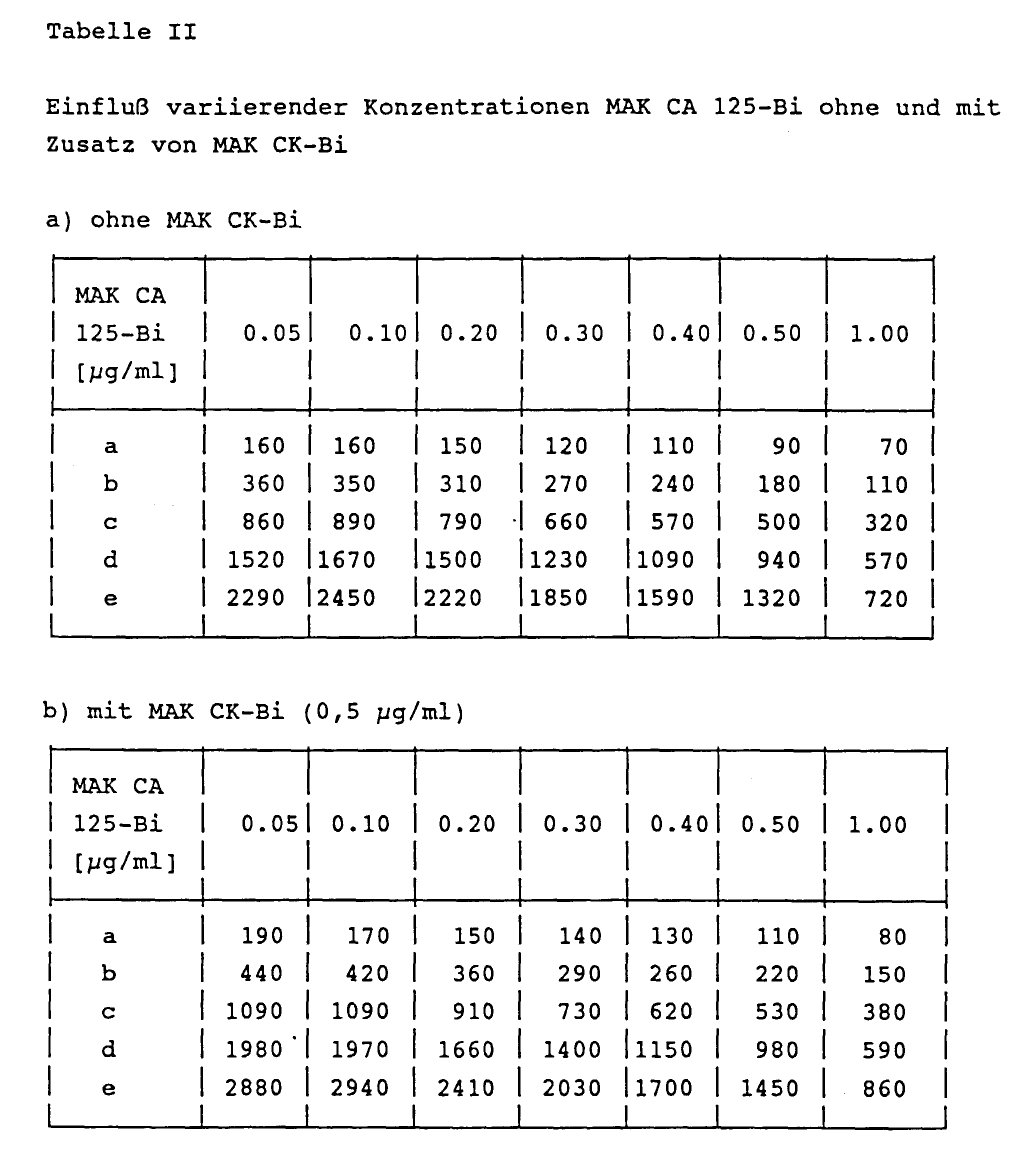

Der Effekt der Zunahme der Signalintensität aufgrund der Anwesenheit des unspezifischen Konjugats MAK CK-Bi wird auch bei variierenden Konzentrationen des Analyt-spezifischen Konjugats MAK CA125-Bi gefunden. Die Wirkung ist besonders dann zu sehen, wenn ein Überschuß von MAK CK-Bi vorliegt.

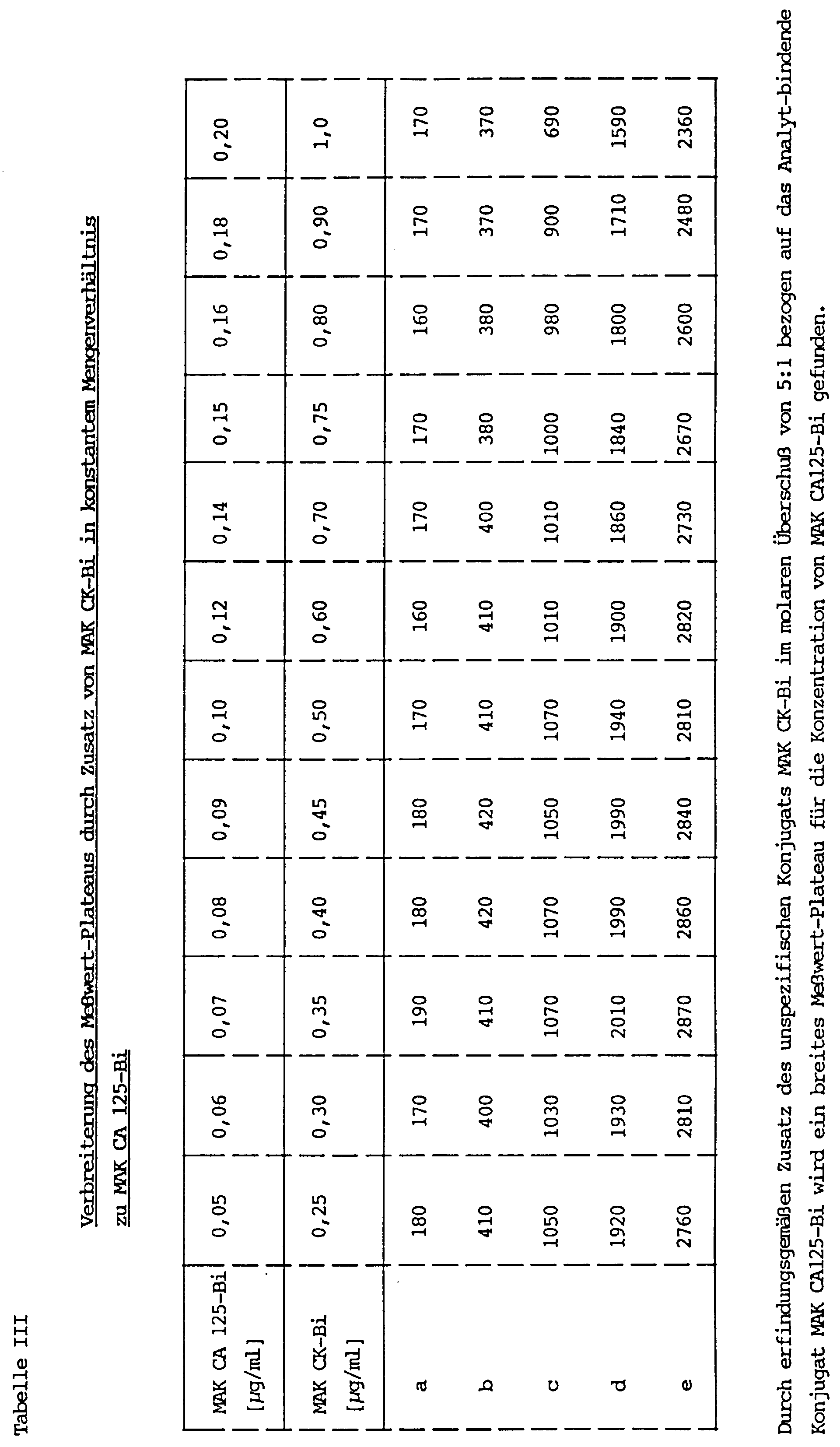

**Tabelle IV**

| **Präzision in der Serie ohne Zusatz von MAK CK-Bi, MAK CA 125-Bi: 0,1 µg/ml** | | | |
|---|---|---|---|
| 20 Einzelbestimmungen | | | |
| | Standardlösung | Humanserum 1 | Humanserum 2 |
| x | 7,02 U/ml | 10,21 U/ml | 18,14 U/ml |
| s | 4,50 U/ml | 4,67 U/ml | 4,46 U/ml |
| V_{K} | 64 % | 46 % | 25 % |

**Tabelle V**

| **Präzision in der Serie 0,1 µg/ml MAK CA 125-Bi (Biotinylierungsgrad 1:10) bei Zusatz von von 0,5 µg/ml MAK CK-Bi** | | | |
|---|---|---|---|
| 20 Einzelbestimmungen | | | |
| | Standardlösung | Humanserum 1 | Humanserum 2 |
| x | 4,92 U/ml | 6,83 U/ml | 12,34 U/ml |
| s | 1,90 U/ml | 2,11 U/ml | 1,26 U/ml |
| V_{K} | 39 % | 31 % | 10 % |

Ein Vergleich der Tabellen IV und V zeigt eine erhöhte Präzision der Bestimmung des Antigens CA-125 bei Zusatz des unspezifischen Konjugats MAK CK-Bi.
- x:: Mittelwert aus 20 Bestimmungen
- s:: Standardabweichung
- V_{K}:: Variationskoeffizient auf Konzentrationsbasis

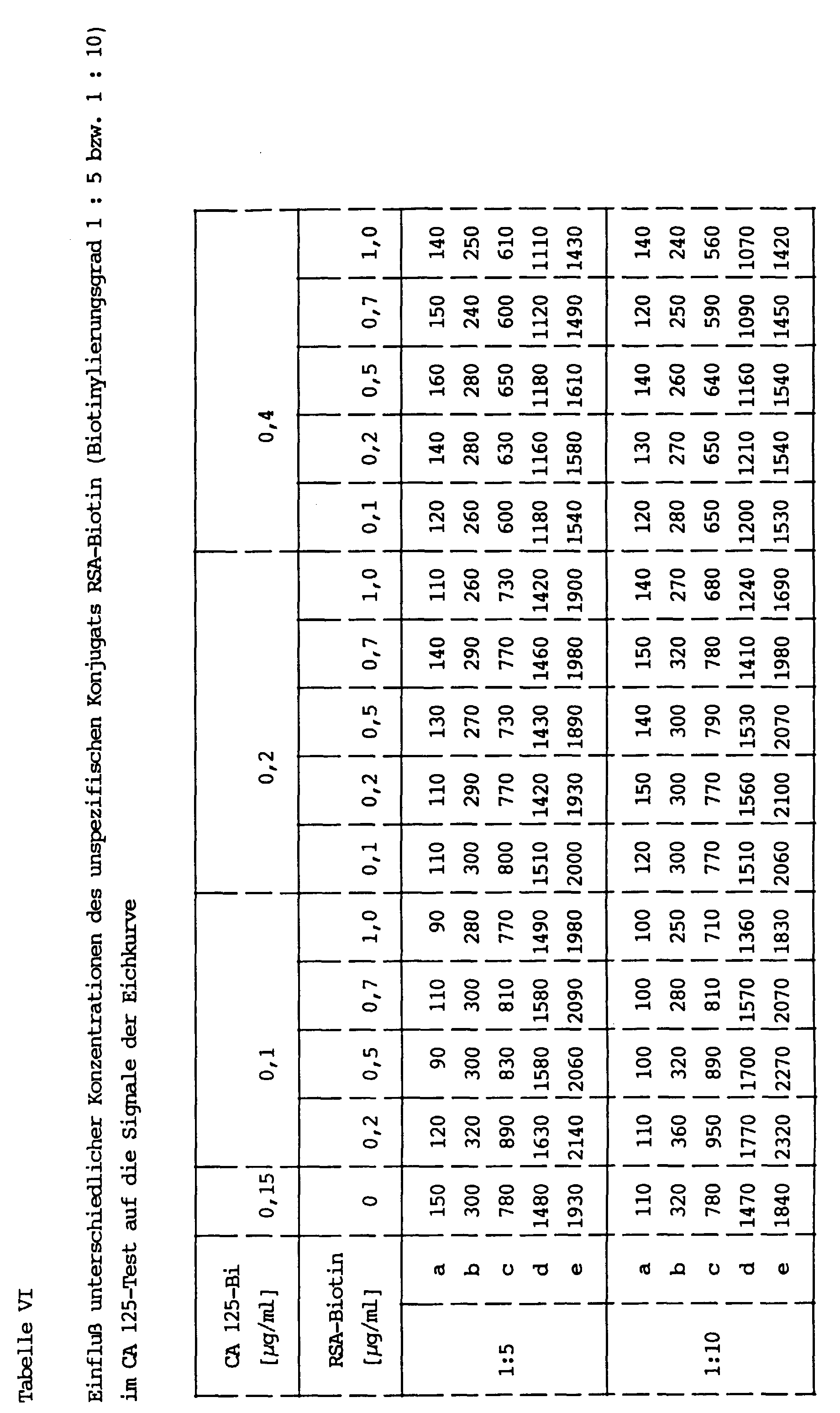

**Tabelle VII**

| **Präzision in der Serie bei Zusatz von RSA-Biotin** | | | | | | | | | |
|---|---|---|---|---|---|---|---|---|---|
| Proben | | ohne Biotin-Konjugat | | | | + 0,5 µg/ml RSA-Biotin (1 : 10) | | | |
| | | Extinktion E | | U/ml | | Extinktion E | | U/ml | |
| | | 10 DB | 20 EB | 10 DB | 20 EB | 10 DB | 20 EB | 10 DB | 20 EB |
| Standard a | x | 0,116 | 0,116 | 4,68 | 5,24 | 0,113 | 0,115 | 2,83 | 3,12 |
| | s | 0,005 | 0,006 | 2,58 | 2,15 | 0,004 | 0,005 | 0,96 | 1,38 |
| | V_{K} | 5 % | 5 % | 55 % | 41 % | 3 % | 4 % | 34 % | 44 % |
| HS 1 | x | 0,160 | 0,160 | 19,24 | 19,22 | 0,162 | 0,161 | 14,34 | 14,44 |
| | s | 0,010 | 0,013 | 3,10 | 3,78 | 0,003 | 0,006 | 0,78 | 1,26 |
| | V_{K} | 6 % | 8 % | 16 % | 20 % | 2 % | 3 % | 5 % | 9 % |
| HS 2 | x | 0,166 | 0,166 | 20,94 | 20,93 | 0,174 | 0,173 | 17,01 | 17,01 |
| | s | 0,010 | 0,011 | 3,04 | 3,33 | 0,003 | 0,008 | 0,64 | 1,73 |
| | V_{K} | 6 % | 7 % | 15 % | 16 % | 2 % | 4 % | 4 % | 10 % |
| HS 3 | x | 0,161 | 0,162 | 19,56 | 20,0 | 0,166 | 0,166 | 15,25 | 15,25 |
| | s | 0,013 | 0,012 | 3,79 | 3,67 | 0,006 | 0,009 | 1,31 | 1,91 |
| | V_{K} | 8 % | 8 % | 19 % | 18 % | 4 % | 5 % | 9 % | 13 % |
| HS 4 | x | 0,174 | 0,174 | 23,39 | 23,39 | 0,184 | 0,184 | 19,32 | 19,32 |
| | s | 0,011 | 0,011 | 3,07 | 3,24 | 0,004 | 0,006 | 0,92 | 1,41 |
| | V_{K} | 6 % | 6 % | 13 % | 14 % | 2 % | 4 % | 5 % | 7 % |
| HS 5 | x | 0,194 | 0,194 | 29,0 | 29,0 | 0,209 | 0,209 | 24,77 | 24,79 |
| | s | 0,009 | 0,010 | 2,42 | 2,68 | 0,004 | 0,007 | 0,92 | 1,48 |
| | V_{K} | 4 % | 5 % | 8 % | 9 % | 2 % | 3 % | 4 % | 6 % |

- HS:: Humanserum
- DB:: Doppelbestimmung
- EB:: Einzelbestimmung

### Beispiel 4

### Bestimmung von Estradiol

100 µl Probe (Humanserum) werden in ein mit Streptavidin beschichtetes Polystyrol-Röhrchen gegeben und mit 70 ng eines biotinylierten Anti-Estradiol-Antikörpers (polyklonal, Herstellung gemäß G. Brown et al. in D. Catty (ed.), Antibodies, Vol. I, A practical approach, IRL-Press, Washington, 1988, 85; Immunisierung mit Estradiol (Boehringer Mannheim GmbH, Katalog Nr. 11 22 789)) ) in 500 µl Inkubationspuffer (100 mmol/l Phosphatpuffer pH 7,0) versetzt und 30 Minuten inkubiert.

Nach Zugabe eines Konjugats aus Peroxidase und einem Anti-Estradiol-Antikörper (Boehringer Mannheim GmbH, Katalog Nr. 11 22 789, 150 mU/ml POD-Aktivität in 500 µl Inkubationspuffer) wird weitere 60 Minuten inkubiert. Nach Absaugen der flüssigen Phase und mehrmaligem Waschen der Röhrchen mit Waschlösung, wird 1 ml Substratlösung zugegeben, 30 Minuten inkubiert und die gebildete Farbe bei 420 nm photometrisch bestimmt. Die Ergebnisse sind aus der Tabelle VIII zu ersehen.

**Tabelle VIII**

| Probe | Variante 1 | | Variante 2 | | Differenz 2/1 |
|---|---|---|---|---|---|
| | mE | % VK | mE | % VK | % |
| 1 | 243 | 8,3 | 302 | 6,6 | 20 |
| 2 | 112 | 8,7 | 158 | 3,4 | 61 |
| 3 | 34 | 13,8 | 59 | 7,0 | 49 |
| 4 | 128 | 5,9 | 147 | 3,7 | 37 |
| Mittelwert | | 9,2 | | 5,2 | 44 |

- Variante 1:: 3 ng Biotin /ml
- Variante 2:: 1.5 µg TRSA-Biotin (1 : 15) /ml
- mE:: Extinktion bei 420 nm in mE

Tabelle VIII zeigt, daß der Zusatz von Biotin allein nicht geeignet ist, die Präzision des Tests wesentlich zu verbessern. Erst der Zusatz eines Konjugats aus hochmolekularem RSA (TRSA, Herstellung entsprechend EP-A 0 269 092) mit Biotin (TRSA-Biotin) führt zu einer Verbesserung der Präzision.

## Patentansprüche

1. Verfahren zur Bestimmung eines Analyten durch einen heterogenen immunologischen Test unter Verwendung eines an einer Festphase fixierten ersten Partners eines Bindepaares, bei dem man eine den Analyten enthaltende Probelösung in Gegenwart der Festphase entweder
(1) mit einem ersten Konjugat, bestehend aus (a) dem zweiten Partner des oben erwähnten Bindepaares und (b) einer ersten, mit dem Analyten immunologisch spezifisch bindefähigen Substanz, oder
(2) mit einem ersten Konjugat, bestehend aus (a) dem zweiten Partner des oben erwähnten Bindepaares und (b) einer ersten, mit dem Analyten immunologisch spezifisch bindefähigen Substanz, sowie mit einer zweiten, mit dem Analyten immunologisch spezifisch bindefähigen markierten Substanz
inkubiert, danach die Phasen trennt, im Falle (1) die flüssige Phase durch eine weitere Lösung, die eine zweite, mit dem Analyten spezifisch bindefähige markierte Substanz enthält, ersetzt und erneut inkubiert und danach abtrennt, und in beiden Fällen die Markierung an der festen Phase oder in der zuletzt abgetrennten flüssigen Phase als Maß für die Analytmenge mißt,
**dadurch gekennzeichnet,**
daß man der flüssigen Phase vor der Inkubation außerdem ein zweites Konjugat aus (a) einem Makromolekül, das nicht mit dem Analyten bindefähig ist, und (b) dem zweiten Partner des oben erwähnten Bindepaares im ]berschuß gegenüber dem ersten Konjugat zusetzt.

2. Verfahren nach Anspruch 1,
**dadurch gekennzeichnet,**
daß man als erste und zweite mit dem Analyten immunologisch spezifisch bindefähige Substanzen Antikörper oder Antikörperfragmente verwendet.

3. Verfahren nach Anspruch 1 oder 2,
**dadurch gekennzeichnet,**
daß man erste und zweite immunologisch bindefähige Substanzen mit gleichartigen Analyt-Bindungsstellen verwendet.

4. Verfahren nach einem der vorhergehenden Ansprüche,
**dadurch gekennzeichnet,**
daß man das zweite Konjugat in einem Überschuß von 2 bis 10 gegenüber dem ersten Konjugat einsetzt.

5. Verfahren nach einem der Ansprüche 1 bis 4,
**dadurch gekennzeichnet,**
daß das zweite Konjugat aus (a) einem Makromolekül, das nicht mit dem Analyten bindefähig ist, und (b) dem zweiten Partner des Bindepaares mehrere Bindungsstellen für den ersten Partner des Bindepaares aufweist.

6. Verfahren nach Anspruch 5,
**dadurch gekennzeichnet,**
daß das zweite Konjugat 5 bis 10 Bindungsstellen für den ersten Partner des Bindepaares aufweist.

7. Reagenz zur Bestimmung eines Analyten durch einen heterogenen immunologischen Test unter Verwendung eines an einer Festphase fixierten ersten Partners eines Bindepaares,
das
(1) ein erstes Konjugat, bestehend aus (a) dem zweiten Partner des oben erwähnten Bindepaares und (b) einer ersten, mit dem Analyten immunologisch spezifisch bindefähigen Substanz,
(2) eine zweite, mit dem Analyten immunologisch spezifisch bindefähige markierte Substanz enthält,
**dadurch gekennzeichnet,**
daß es
(3) ein zweites Konjugat, bestehend aus (a) einem Makromolekül, das nicht mit dem Analyten bindefähig ist, und (b) dem zweiten Partner des oben erwähnten Bindepaares enthält.

8. Reagenz nach Anspruch 7,
**dadurch gekennzeichnet,**
daß die ersten und zweiten, immunologisch spezifisch bindefähigen Substanzen Antikörper oder Antikörperfragmente darstellen.

9. Reagenz nach Anspruch 7 oder 8,
**dadurch gekennzeichnet,**
daß die ersten und zweiten, immunologisch spezifisch bindefähigen Substanzen gleichartige Analyt-Bindungsstellen aufweisen.

10. Reagenz nach einem der Ansprüche 7 bis 9,
**dadurch gekennzeichnet,**
daß das zweite Konjugat aus (a) einem Makromolekül, das nicht mit dem Analyten bindefähig ist, und (b) dem zweiten Partner des Bindepaares mehrere Bindungsstellen für den ersten Partner des Bindepaares aufweist.

## Claims

1. Method for the determination of an analyte by a heterogeneous immunological test using a first partner of a binding pair which is immobilized on a solid phase in which a sample solution containing the analyte is incubated in the presence of the solid phase with either
(1) a first conjugate consisting of (a) the second partner of the above-mentioned binding pair and (b) a first substance capable of specific immunological binding to the analyte or
(2) a first conjugate consisting of (a) the second partner of the above-mentioned binding pair and (b) a first substance capable of specific immunological binding to the analyte as well as with a second labelled substance capable of specific immunological binding to the analyte,
afterwards the phases are separated, in case (1) the liquid phase is replaced by a further solution containing a second labelled substance capable of binding specifically to the analyte and it is again incubated and subsequently separated, and in both cases the label on the solid phase or in the liquid phase which was separated last is measured as a measure of the amount of analyte
**wherein**
a second conjugate of (a) a macromolecule which is not capable of binding to the analyte and (b) the second partner of the above-mentioned binding pair is additionally added to the liquid phase in an excess over the first conjugate.

2. Method as claimed in claim 1,
**wherein**
antibodies or antibody fragments are used as the first and second substances which are capable of specific immunological binding to the analyte.

3. Method as claimed in claim 1 or 2,
**wherein**
first and second immunologically bindable substances with the same type of analyte binding sites are used.

4. Method as claimed in one of the previous claims,
**wherein**
the second conjugate is used in an excess of 2 to 10 over the first conjugate.

5. Method as claimed in one of the claims 1 to 4,
**wherein**
the second conjugate consisting of (a) a macromolecule which is not capable of binding to the analyte and (b) the second partner of the binding pair has several binding sites for the first partner of the binding pair.

6. Method as claimed in claim 5,
**wherein**
the second conjugate has 5 to 10 binding sites for the first partner of the binding pair.

7. Reagent for the determination of an analyte by a heterogeneous immunological test using a first partner of a binding pair which is immobilized on a solid phase, which contains
(1) a first conjugate consisting of (a) the second partner of the above-mentioned binding pair and (b) a first substance capable of specific immunological binding to the analyte
(2) a second labelled substance capable of specific immunological binding to the analyte,
**wherein**
it contains
(3) a second conjugate consisting of (a) a macromolecule which is not capable of binding to the analyte and (b) the second partner of the above-mentioned binding pair.

8. Reagent as claimed in claim 7,
**wherein**
the first and second specific immunologically bindable substances constitute antibodies or antibody fragments.

9. Reagent as claimed in claim 7 or 8,
**wherein**
the first and second specific immunologically bindable substances have the same type of analyte binding sites.

10. Reagent as claimed in one of the claims 7 to 9
**wherein**
the second conjugate consisting of (a) a macromolecule which is not capable of binding to the analyte and (b) the second partner of the binding pair has several binding sites for the first partner of the binding pair.

## Revendications

1. Procédé de détermination d'un analyte par un test immunologique hétérogène au moyen d'un premier partenaire d'une paire de liaison fixé à une phase solide, dans lequel on incube une solution échantillon contenant l'analyte en présence de la phase solide
(1) avec un premier conjugué consistant en (a) le deuxième partenaire de la paire de liaison citée ci-dessus et (b) une première substance capable de se lier spécifiquement avec l'analyte par voie immunologique, ou
(2) avec un premier conjugué consistant en (a) le deuxième partenaire de la paire de liaison citée ci-dessus et (b) une première substance capable de se lier spécifiquement avec l'analyte par voie immunologique, ainsi qu'avec une deuxième substance marquée capable de se lier spécifiquement avec l'analyte par voie immunologique,
puis on sépare les phases, dans le cas (1) on remplace la phase liquide par une autre solution qui contient une deuxième substance marquée capable de se lier spécifiquement avec l'analyte et on incube de nouveau puis on sépare, et dans les deux cas on mesure le marquage au niveau de la phase solide ou dans la phase liquide séparée en dernier lieu en tant que mesure de la quantité d'analyte,
caractérisé en ce que l'on ajoute en outre à la phase liquide avant l'incubation un deuxième conjugué de (a) une macromolécule qui n'est pas capable de se lier avec l'analyte et de (b) le deuxième partenaire de la paire de liaison citée ci-dessus en excès par rapport au premier conjugué.

2. Procédé selon la revendication 1, caractérisé en ce que l'on utilise comme première et deuxième substances capables de se lier spécifiquement avec l'analyte par voie immunologique des anticorps ou des fragments d'anticorps.

3. Procédé selon la revendication 1 ou 2, caractérisé en ce que l'on utilise des première et deuxième substances capables de se lier par voie immunologique ayant des sites de liaison avec l'analyte de même type.

4. Procédé selon l'une des revendications précédentes, caractérisé en ce que l'on utilise le deuxième conjugué en un excès de 2 à 10 par rapport au premier conjugué.

5. Procédé selon l'une des revendications 1 à 4, caractérisé en ce que le deuxième conjugué de (a) une macromolécule qui n'est pas capable de se lier avec l'analyte et de (b) le deuxième partenaire de la paire de liaison comporte plusieurs sites de liaison pour le premier partenaire de la paire de liaison.

6. Procédé selon la revendication 5, caractérisé en ce que le deuxième conjugué comporte 5 à 10 sites de liaison pour le premier partenaire de la paire de liaison.

7. Réactif de détermination d'un analyte par un test immunologique hétérogène au moyen d'un premier partenaire d'une paire de liaison fixé à une phase solide, qui contient
(1) un premier conjugué consistant en (a) le deuxième partenaire de la paire de liaison citée ci-dessus et (b) une première substance capable de se lier spécifiquement avec l'analyte par voie immunologique,
(2) une deuxième substance marquée capable de se lier spécifiquement avec l'analyte par voie - immunologique,
caractérisé en ce qu'il contient
(3) un deuxième conjugué consistant en (a) une macromolécule qui n'est pas capable de se lier avec l'analyte et en (b) le deuxième partenaire de la paire de liaison citée ci-dessus.

8. Réactif selon la revendication 7, caractérisé en ce que les première et deuxième substances capables de se lier spécifiquement par voie immunologique sont des anticorps ou des fragments d'anticorps.

9. Réactif selon la revendication 7 ou 8, caractérisé en ce que les première et deuxième substances capables de se lier spécifiquement par voie immunologique comportent des sites de liaison avec l'analyte de même type.

10. Réactif selon l'une des revendications 7 à 9, caractérisé en ce que le deuxième conjugué de (a) une macromolécule qui n'est pas capable de se lier avec l'analyte et de (b) le deuxième partenaire de la paire de liaison comporte plusieurs sites de liaison pour le premier partenaire de la paire de liaison.
